# FASCICULE DE BREVET EUROPEEN

(11) **EP 3 868 442 B1**
(45) Date de publication et mention de la délivrance du brevet: **07.02.2024**
(21) Numéro de dépôt: 21157905.7
(22) Date de dépôt: 18.02.2021
(51) Int. Cl.: A61K 31/734, A61K 31/4439, A61K 31/341, A61K 31/24, A61K 31/05, A61P 1/04, A61P 27/16

(54) **COMPOSITION POUR LE TRAITEMENT DES VERTIGES PERIPHERIQUES**
ZUSAMMENSETZUNG FÜR DIE BEHANDLUNG VON PERIPHEREN SCHWINDELANFÄLLEN
COMPOSITION FOR THE TREATMENT OF PERIPHERAL VERTIGO

(30) Priorité: 19.02.2020 FR 2001662
(43) Date de publication de la demande: 25.08.2021
(73) Titulaire: Amsellem, Jean-Paul, 31000 Toulouse (FR)
(72) Inventeur: Amsellem, Jean-Paul, 31000 Toulouse (FR)
(74) Mandataire: Aquinov

(56) Documents cités:
- WO-A1-2014/165823
- CN-B- 103 494 792
- US-A1- 2007 281 015
- US-A1- 2018 140 630
- KUNG YU-MIN ET AL: "Recent Advances in the Pharmacological Management of Gastroesophageal Reflux Disease", DIGESTIVE DISEASES AND SCIENCES, SPRINGER NEW YORK LLC, US, vol. 62, no. 12, 6 novembre 2017 (2017-11-06), pages 3298-3316, XP036367030, ISSN: 0163-2116, DOI: 10.1007/S10620-017-4830-5 [extrait le 2017-11-06]
- WANG WEI: "THE ROLE OF TRIMEBUTINE IN THE TREATMENT OF ELDERLY PATIENTS WITH REFLEX ESOPHAGITIS", GASTROENTEROLOGY, vol. 152, no. 5, Suppl. 1, avril 2017 (2017-04), page S697, XP002800715, & DIGESTIVE DISEASE WEEK (DDW); CHICAGO, IL, USA; MAY 06 -09, 2017
- HEE-YOUNG KIM: "Vertigo due to Eustachian Tube Dysfunction", ARCHIVES OF OTORHINOLARYNGOLOGY HEAD & NECK SURGERY, 1 janvier 2017 (2017-01-01), XP055740805, DOI: 10.24983/scitemed.aohns.2017.00017
- HEE-YOUNG KIM: "Reciprocal Causal Relationship between Laryngopharyngeal Reflux and Eustachian Tube Obstruction", JOURNAL OF OTOLARYNGOLOGY-ENT RESEARCH, vol. 2, no. 6, 15 juin 2015 (2015-06-15), XP055740820, DOI: 10.15406/joentr.2015.02.00046
- EDITA VILIUSYTE: "Associations between peripheral vertigo and gastroesophageal reflux", MEDICAL HYPOTHESES, vol. 85, 1 janvier 2015 (2015-01-01), pages 333-335, XP055740620,
- LOU YUE ET AL: "Efficacy of BPPV diagnosis and treatment system for benign paroxysmal positional vertigo", AMERICAN JOURNAL OF OTOLARYNGOLOGY, W.B. SAUNDERS, PHILADELPHIA, PA, US, vol. 41, no. 3, 4 février 2020 (2020-02-04), XP086175335, ISSN: 0196-0709, DOI: 10.1016/J.AMJOTO.2020.102412 [extrait le 2020-02-04]
- ZAMERGRAD M V ET AL: "Common Causes of Vertigo and Dizziness in Different Age Groups of Patients", BIONANOSCIENCE, SPRINGER US, BOSTON, vol. 7, no. 2, 19 octobre 2016 (2016-10-19), pages 259-262, XP036230893, ISSN: 2191-1630, DOI: 10.1007/S12668-016-0351-5 [extrait le 2016-10-19]
- DATABASE BIOSIS [Online] BIOSCIENCES INFORMATION SERVICE, PHILADELPHIA, PA, US; April 2017 (2017-04), WANG WEI: "THE ROLE OF TRIMEBUTINE IN THE TREATMENT OF ELDERLY PATIENTS WITH REFLEX ESOPHAGITIS", Database accession no. PREV201700639623 & GASTROENTEROLOGY, vol. 152, no. 5, Suppl. 1, April 2017 (2017-04), page S697, DIGESTIVE DISEASE WEEK (DDW); CHICAGO, IL, USA; MAY 06 -09, 2017 ISSN: 0016-5085(print)

## Description

### Domaine technique

L'invention concerne la prévention et/ou le traitement des vertiges périphériques. En particulier, l'invention se rapporte à une nouvelle composition comprenant au moins un alginate, et au moins un inhibiteur de la pompe à pour son utilisation dans la prévention et/ou le traitement des vertiges positionnels paroxystiques bénins positionnels.

### Etat de l'art

Les vertiges représentent un motif fréquent de consultation médicale, les causes sont nombreuses et nécessitent un diagnostic précis effectué par un praticien. La plupart des troubles sont sans gravité et le traitement dépend essentiellement du diagnostic.

Un vertige est une sensation désagréable dans laquelle le patient a l'impression que tout tourne autour de lui-même, accompagné d'une sensation de perte d'équilibre. Il doit être différentié d'un malaise, associé ou non, à une perte de conscience.

Les symptômes associés aux vertiges peuvent être transitoires (quelques minutes à quelques heures) ou durables. Ils peuvent éventuellement s'accompagner de nausées et de vomissements voire, dans certains cas, d'une perte de l'audition. Des troubles de la vue peuvent également se manifester tel qu'une vision double (diplopie). Le vertige se traduit également par la présence d'un nystagmus (secousse involontaire des yeux directement liée aux phénomènes vertigineux). Les vertiges peuvent survenir à tout âge mais ils sont plus fréquents lors de la deuxième moitié de la vie. Les personnes âgées souffrent majoritairement de troubles de l'équilibre mais peuvent souffrir également de véritables vertiges.

Les étiologies sont nombreuses et variées, mais les vertiges sont souvent associés à un dysfonctionnement de l'oreille interne. Parmi les vertiges les plus fréquents, il faut noter le vertige positionnel paroxystique bénin (VPPB), la maladie de Ménière et éventuellement des infections (labyrinthite virale ou névrite vestibulaire). Des médicaments, drogues, peuvent également engendrer des sensations vertigineuses, des sensations d'instabilité ou de déséquilibre. Enfin certains vertiges peuvent également être responsables de troubles visuels. Hee-Young Kim et al. 2017 ; Hee-Young Kim et al. 2015 ; Edita Viliusyte et al. 2015 ont constaté une concomitance clinique entre le reflux gastro-oesophagique et certains vertiges. Cependant aucune explication physiopathologique n'a été apporté permettant de démontrer un quelconque lien entre les deux pathologies. De plus, les vertiges cités ne sont pas des vertiges positionnels paroxystiques bénins, aucun ne décrit la présence de petits cristaux de carbonate de calcium (CaCO3) dans les canaux semi-circulaires.

Le vertige positionnel paroxystique bénin est le plus fréquent et est majoritairement déclenché par un changement de position rapide de la tête, notamment lors du passage de la position couchée à une position levée et inversement ou lors d'une inclinaison brutale de la tête. Ce vertige rotatoire, bénin, se déclenche quelques secondes après le mouvement et dure moins d'une minute. Il peut être accompagné de nausées, rarement de vomissement.

Le vertige de position paroxystique bénin est dû à une mauvaise distribution des petits cristaux de carbonate de calcium (CaCO3) dit otolithes habituellement répartis de manière homogène dans les canaux semi-circulaires de l'oreille interne. Lors de l'apparition des symptômes, les otolithes s'accumulent dans un seul canal, l'oreille interne perçoit alors un signal anormalement fort qui est en contradiction avec ce que perçoivent les yeux et le corps. Cette discordance déclenche le vertige. (Rapport de la Société Française d'Oto-Rhino-Laryngologie et de la Chirurgie de la Face et du Cou, (2007)).

Le traitement du vertige de position paroxystique bénin repose sur des manipulations de kinésithérapie (dites manoeuvres libératoires, manoeuvre de Sémont ou manoeuvre d'Epley) (Vitte E. et al. 1995 (2ème éd.) Courtat Ph., Peytral Cl., Elbaz P. - Masson éditeur Paris 1 La kinésithérapie en ORL). Le patient est allongé et le médecin effectue diverses rotations de la tête du patient entrecoupées de pause. Les otolithes sont ainsi débloqués et répartis de nouveau uniformément dans les canaux semi-circulaires de l'oreille interne, le soulagement est alors immédiat. Néanmoins, le patient doit ensuite rester assis pendant 24 heures (y compris pour dormir). Il peut être nécessaire de répéter ces manoeuvres au cours de plusieurs séances. De plus, ces manipulations de la tête sont assez violentes. Or, la plupart des patients sont des personnes âgées, dites fragiles dont les manipulations ne sont pas adaptées à l'âge du patient. Les patients peuvent également appréhender ces séances de kinésithérapie.

A ce jour, seule des solutions non-médicamenteuses sont donc efficaces et permettent de traiter les vertiges positionnels paroxystiques bénins. Aucun traitement médicamenteux n'a prouvé son efficacité, que ce soit pour prévenir et/ou traiter lesdits vertiges. Eventuellement, certains traitements médicamenteux peuvent être prescrit pour soulager les symptômes, tel que des médicaments anti-vertigineux, notamment l'acétylleucine, dont le mécanisme d'action n'a pas été démontré, ou la bétahistine plus volontiers prescrite pour les pathologies pressionnelles de l'oreille interne ou des médicaments pour lutter contre la nausée. Néanmoins, leur faible efficacité a été montrée dans plusieurs études.

Le VPPB est le plus fréquent des vertiges et peut être ressenti comme très anxiogène. Le VPPB n'est pas anodin car certains patients peuvent vivre une importante limitation d'activités. Celui-ci s'accompagne de troubles de l'équilibre, voire de chute.

En Allemagne, selon Brevern et al., la prévalence à vie du VPPB est de 2,4% (Von Brevern et al., 2007). L'incidence annuelle dans le même pays est approximativement de 3,1%, et la prévalence est de 22,9% (Neuhauser et al., 2008). Aux États-Unis, 5,6 millions de visites cliniques ont été réalisées pour un vertige comme motif initial de consultation, et entre 17 et 42% des patients ayant un vertige ont reçu un diagnostic de VPPB (American Academy of Otolaryngology-Head and Neck Surgery Foundation, 2008).

De façon générale, le VPPB est sous-évalué et est associé à un risque augmenté de chute et à une mauvaise qualité de vie (Alghadir, 2013). Selon une étude épidémiologique nord-américaine, les dépenses liées au traitement du VPPB ont atteint la somme de 2000 dollars par patient. La plupart de ces coûts n'étaient pas nécessaires et étaient associés à des mauvais diagnostics et des traitements inefficaces, en particulier médicamenteux (Li et al. 2000).

Aussi, la prévention et/ou le traitement des VPPB est un problème connu depuis nombreuses années sans solution thérapeutique médicamenteuse efficace. Seul le traitement de référence, soit les manoeuvres dites libératoires, permet de traiter les vertiges. Toutefois, dans certains cas, il apparait que le traitement de référence n'est pas suffisant pour traiter les vertiges périphériques récidivants en particulier des vertiges positionnels bénins récidivants.

Par conséquent, il existe un réel besoin pour un traitement alternatif préventif et/ou curatif permettant de surmonter ces inconvénients.

Actuellement, aucun traitement systémique n'a donc démontré son efficacité pour traiter les vertiges périphériques ou ces symptômes et il est par conséquent d'intérêt de développer une nouvelle composition capable de surmonter les inconvénients de l'art antérieur, qui soit efficace et qui permettent de s'affranchir des séances de kinésithérapie mais également d'éviter la récidive. C'est l'objectif de la présente invention.

### Résumé de l'invention

Or, l'invention propose enfin de résoudre ce problème. Ainsi, l'invention concerne un traitement médicamenteux pour prévenir et/ou traiter les vertiges positionnels paroxystiques bénins qui soit simple, efficace.

De façon fortuite, les inventeurs ont constaté un lien entre reflux gastro-oesophagien et les vertiges positionnels paroxystiques bénins. L'hyperacidité gastrique, peu importe son origine, éventuellement associée à des spasmes de l'estomac, ou simplement par induite par la position déclive en position couchée, génère un reflux gastro-cesophago-pharyngien pouvant remonter dans le rhinopharynx, irritant celui-ci, et plus haut encore, par les trompes d'Eustache, l'oreille.

Les inventeurs ont découvert que le reflux gastro-oesophagien aboutissait à une modification du métabolisme de l'oreille interne. Un pH très acide modifie le métabolisme labyrinthique et perturbe le cycle otolithique, induisant une désagrégation des petits cristaux de carbonate de calcium (CaCO₃) ou otolithes aboutissant un relargage otolithique dans les canaux semi-circulaires. Les otolithes relargués de façon hétérogène dérèglent alors l'oreille interne, organe de l'équilibre aboutissant à des informations erronées responsables d'un conflit neurosensoriel à l'origine de la survenue de ces vertiges.

Aussi, pour répondre à son objectif, l'invention propose d'utiliser une composition capable de prévenir et/ou traiter les vertiges positionnels paroxystiques bénins. A cet effet l'invention, vise une nouvelle composition comprenant au moins un alginate et au moins un inhibiteur de la pompe à protons pour son utilisation dans la prévention et/ou le traitement des vertiges positionnels paroxystiques bénins (VPPB).

Avantageusement une telle composition permet une action combinée, synergique et efficace sur ces vertiges mais également sur le reflux gastro-oesophagien à l'origine de ces vertiges.

Ainsi, l'invention concerne également ladite composition pour son utilisation comme médicament, préférentiellement dans la prévention et/ou le traitement des vertiges périphériques, plus préférentiellement les vertiges positionnels paroxystiques bénins et/ou pour son utilisation dans la prévention et/ou le traitement du reflux gastro-oesophagien.

D'autres caractéristiques et avantages ressortiront de la description détaillée de l'invention et des exemples qui vont suivre.

### Définitions

Par « alginate », on entend au sens de l'invention un acide alginique est ses dérivés (N° CAS : 9005-32-7). En particulier, il s'agit de polysaccharides obtenus à partir d'algues brunes telles que les laminaires ou les fucus. Un alginate est formé de deux monomères liés ensemble : l'acide manuronique et l'acide galuronique. Les alginates sont notamment utilisés comme additifs alimentaires pour leurs qualités épaississantes et gélifiantes, notamment sous le n° E400, ainsi qu'en pharmacie dans l'encapsulation de certains médicaments.

Par « inhibiteur de la pompe à protons » ou « IPP », on entend au sens de l'invention des molécules ayant une action sur la pompe à protons permettant de réduire l'acidité gastrique. Leur utilisation est donc bien connue dans le traitement et la prévention des reflux gastro-oesophagien et de l'oesophagite liée au RGO.

Par « antihistaminique H2 », on entend au sens de l'invention des antagonistes des récepteurs à l'histamine de type 2. Ils agissent au niveau des cellules pariétales de l'estomac pour diminuer sa sécrétion acide et sont utilisés comme médicaments dans le traitement des reflux gastro-oesophagien ou des ulcères gastro-duodénaux. Ils ont été largement supplantés par l'arrivée sur le marché des IPP du fait d'une meilleure efficacité dans ces indications.

Par « antispasmodique » ou « spasmolytique », on entend au sens de l'invention des molécules ou médicaments permettant de lutter contre les spasmes musculaires principalement localisés au niveau digestif.

Par « gastro-oesophagite spastique » on entend au sens de l'invention une inflammation de la muqueuse gastro-oesophagienne due à une hyperacidité déclenchant des spasmes essentiellement gastriques, pouvant générer une remontée des sucs gastriques et donc un reflux gastro-oesophagien d'origine spastique.

Par « ototoxicité », on entend au sens de l'invention toute substance chimique exogène qui est toxique pour les structures de l'oreille interne, que ce soit la cochlée, le système vestibulaire ou les deux.

Par « patient » ou « sujet », on entend au sens de l'invention un sujet qui peut être un être humain ou un animal, de préférence un être humain ou un mammifère. De préférence, le sujet est un patient humain, quel que soit son âge ou son sexe, plus préférentiellement l'âge moyen est de 50 à 70 ans et la prévalence augmente chez les sujets âgés. Les nouveau-nés, les nourrissons et les enfants sont également inclus.

Par « pathologie lithiasique labyrinthique », on entend au sens de l'invention un déplacement otolithique dû à une pathologie lithiasique canalaire c'est-à-dire la présence d'un calcul au niveau d'un canal semi-circulaire du labyrinthe.

Par « reflux gastro-oesophagien » on entend au sens de l'invention, un reflux gastro-oesophagien ou RGO indifféremment de son origine. Son origine peut être soit mécanique, c'est-à-dire que le RGO est induit par des spasmes de l'estomac, soit passive, c'est-à-dire que le RGO est induit par la position déclive, allongée, qui provoque la remontée du bol gastrique simplement par le fait de la gravité vers le pharynx.

Par « traitement » ou « traiter », on entend au sens de l'invention l'action d'inverser, d'atténuer, d'inhiber la progression ou de prévenir le trouble ou l'état auquel ce terme s'applique. Préférentiellement, on entend l'action d'inverser, d'atténuer, d'empêcher la progression ou de prévenir les symptômes liés aux vertiges positionnels paroxystiques bénins.

Par « vertiges périphériques » on entend au sens de l'invention un vertige, quelle que soit sa cause, d'origine périphérique excluant ainsi les vertiges centraux. Par vertige central, on entend un vertige dont l'origine est le cerveau, par exemple une tumeur du cerveau. Les vertiges périphériques sont strictement d'ordre fonctionnel et excluent les vertiges périphériques d'origine organique. Préférentiellement, les vertiges périphériques sont des vertiges périphériques récidivants. Par vertiges récidivant on entend au sens de l'invention des vertiges qui surviennent de manière régulière, y compris après des séances de kinésithérapie (manoeuvre dites libératoires).

Par « vertige de position paroxystique bénin » ou « vertiges positionnels paroxystiques bénins » on entend au sens de l'invention indifféremment un « vertige paroxystique bénin » ou « vertige positionnel » ou « VPPB ». Il s'agit d'un vertige rotatoire vrai, déclenché par les changements de positions de la tête, durant moins de 60 secondes, associés à un nystagmus, sans signe cochléaire ni neurologique. Il disparait spontanément dans 30 % des cas sous sept jours. Il implique un ou plusieurs canaux semi-circulaires et provoque différents types de symptômes associés (nausées, vomissements). L'identification du ou des canaux incriminés ainsi que la résolution des symptômes est essentiellement clinique, et requiert la maîtrise de manipulation et de gestes de kinésithérapie, à la fois précis et complexes. Préférentiellement, les vertiges périphériques sont des vertiges positionnels paroxystiques bénins récidivants.

### Description détaillée de l'invention

L'invention a donc pour objet une composition comprenant :
- au moins un alginate, et
- au moins un inhibiteur de la pompe à protons, pour son utilisation dans la prévention et/ou le traitement de vertiges positionnels paroxystiques bénins.

Ainsi, l'invention comprend au moins un alginate et au moins un composé choisi parmi un inhibiteur de la pompe à protons.

Le ou les alginates présents dans la composition selon l'invention sont choisis parmi l'alginate de Sodium, Calcium, Potassium, Magnésium, Aluminium et Fer. Préférentiellement il s'agit d'un alginate de Sodium. Selon un mode de réalisation particulier de l'invention, l'alginate peut être remplacé par n'importe lequel des autres épaississants alimentaires acceptables dans une composition pharmaceutique (https://www.les-additifs-alimentaires.com/liste-epaississants-loi.php). Dans le contexte de l'invention, il est préférable que le ou les alginates soi(ent) présent(s) en quantité plus importante que l'inhibiteur de la pompe à protons pour provoquer un épaississement du liquide gastrique suffisant.

Lorsque la composition comprend au moins un alginate et au moins un inhibiteur de la pompe à protons, la dose d'alginate par prise est comprise entre 50 mg et 1000 mg et la dose d'inhibiteur de la pompe à proton par prise est comprise entre 5 mg et 80 mg.

L'inhibiteur de la pompe à protons peut être choisi notamment parmi l'oméprazole, l'esoméprazole, le lanzoprazole, le pantoprazole et le rabéprazole. Préférentiellement, la composition comprend au moins un alginate et l'oméprazole.

Lorsque la composition comprend l'esoméprazole, la dose par prise est comprise entre 10 mg et 80 mg. Lorsque la composition comprend le lanzoprazole, la dose par prise est comprise entre 15 mg et 60 mg. Lorsque la composition comprend l'oméprazole, la dose par prise est comprise entre 5 mg et 40 mg.

La composition selon l'invention peut comprendre d'autres molécules, principes actifs ou excipients, en plus du ou des alginate(s), du ou des inhibiteur(s) de la pompe à protons.

La composition peut en effet notamment comprendre également au moins un antiacide à action locale et/ou un pansement gastrique.

L'antiacide à action locale est un traitement symptomatique qui est administré lors de la survenance des troubles gastriques. Il est particulièrement indiqué lors des remontés acides et permet de diminuer ou de neutraliser l'acidité du suc gastrique déjà sécrété. L'antiacide à action locale est préférentiellement choisi parmi le Maalox, le Phosphalugel, le Rennie, le Neutricid, le Rocgel et le Xolaam.

Le pansement gastrique est également un traitement symptomatique et vise à protéger la muqueuse gastrique lésée ou risquant de l'être. Il sert de barrière protectrice entre la muqueuse lésée et les sucs digestifs (dont l'acide chlorhydrique) ainsi que les aliments. Le pansement gastrique peut-être choisi notamment parmi le Pepsane, le Polysilane, le Gastropulgite, le Neutrose et le Gelox.

La composition selon l'invention peut également comprendre au moins un excipient pharmaceutique acceptable qui va conférer au médicament des qualités de stabilité, forme, dissolution, ciblage, goût, couleur, esthétique. Par « excipient pharmaceutique », on entend au sens de l'invention un composé ou une combinaison de composés ne provoquant pas de réactions secondaires et qui permet par exemple la facilitation de l'administration du composé actif, l'augmentation de sa durée de vie et/ou de son efficacité dans l'organisme, l'augmentation de sa solubilité en solution ou encore l'amélioration de sa conservation ou de sa biodisponibilité. Ces excipients pharmaceutiquement acceptables sont bien connus et seront adaptés par l'homme de l'art en fonction de la nature et du mode d'administration de la composition.

La composition selon peut se présenter sous toute forme galénique, solide, liquide ou gel, lorsqu'elle ne comprend pas d'inhibiteur de la pompe à protons. Lorsqu'elle comprend au moins un inhibiteur de la pompe à protons, la composition selon l'invention peut se présenter sous toute forme galénique sauf la forme liquide, car cette forme ne permet pas d'avoir un mélange homogène de l'alginate et de l'inhibiteur de la pompe à protons, tel que l'oméprazole par exemple.

La composition selon l'invention se présente préférentiellement sous forme de poudre et/ou de granules et/ou de microgranules. Très préférentiellement elle est conditionnée dans un stick pack comprenant de la poudre, des granules, des microgranules ou leurs mélanges. Les microgranules sont particulièrement préférées pour les IPP pour les rendre gastro résistants car ils sont susceptibles d'être détruits en pH acide dans l'estomac. Les microgranules selon l'invention ont préférentiellement un diamètre inférieur ou égal à 3 mm, de préférence inférieur ou égal à 1 mm.

La composition sous forme de poudre peut être obtenue après le mélange d'une poudre d'alginate et d'une poudre d'inhibiteur de la pompe à protons tel que l'oméprazole. Il est bien connu que l'acide alginique se présente sous forme d'une poudre cristalline ou amorphe, blanche ou brun-jaune pâle et l'oméprazole se présente également sous forme de poudre. Ainsi, l'homme du métier de par ses connaissances générales dans la préparation de compositions pharmaceutiques sera en mesure de préparer une composition selon l'invention sous forme d'une poudre homogène. Bien entendu, l'homme du métier à partir de ses connaissances générales est également en mesure de préparer à partir d'une poudre un comprimé d'alginate et/ou inhibiteur de la pompe à protons ou une gélule d'alginate et/ou inhibiteur de la pompe à protons.

Préférentiellement, la composition selon l'invention comprenant les différents principes actifs choisis parmi un alginate, un inhibiteur de la pompe à protons, et leur mélange se présente sous une seule forme galénique, c'est-à-dire que l'ensemble des principes actifs sont présents dans une seule et même forme médicamenteuse, préférentiellement sous forme de poudre.

A partir d'une poudre d'alginate et d'une poudre d'inhibiteur de la pompe à protons il est possible de procéder à leur mélange pour obtenir la composition selon l'invention. Une telle méthode de préparation peut comprendre les étapes suivantes :
- mélange des poudres de départ,
- pulvérisation des poudres, et
- tamisage des poudres.

L'étape de pulvérisation et tamisage permet d'obtenir des poudres ayant une densité et une taille de particules similaires, nécessaire à l'obtention d'un mélange homogène.

L'homme du métier est en mesure de déterminer le mélangeur le plus adapté, on peut notamment citer l'utilisation d'un mélangeur à cuve mobile, d'un mélangeur à cuve fixe, d'un mélangeur malaxeurs, d'un mélangeur type pétrin etc.

Selon un autre mode de réalisation, la composition selon l'invention est sous forme de granules. La composition sous forme de granules peut être préparée à partir de la composition sous forme de poudre homogène telle que décrite précédemment. Les granules sont obtenues soit par compression direct des poudres, soit par granulation humide, soit par granulation sèche. L'homme du métier sera en mesure de déterminer le procédé de granulation le plus adapté pour obtenir la composition selon l'invention.

Selon un autre mode de réalisation, la composition selon l'invention est sous forme de granule et poudre. Ladite composition peut être préparée à partir d'un mélange de poudre et de granule permettant d'obtenir une composition comprenant un mélange de poudre et granule.

Selon un autre mode de réalisation, la composition selon l'invention est sous forme de microgranules. Les microgranules sont particulièrement adaptées dans le contexte de l'invention afin d'être plus stables, plus résistantes, notamment gastro-résistantes. Les microgranules sont préparées selon un procédé similaire à celui des granules. Bien entendu, l'homme du métier sait adapter le procédé de préparation et le dispositif de granulation afin d'obtenir des microgranules d'un diamètre inférieur ou égal à 3 mm ou 1 mm.

Selon un autre mode de réalisation, la composition selon l'invention est sous forme d'un mélange de poudre et/ou granules et/ou microgranules. Préférentiellement, la composition est sous forme de poudre d'alginate et de microgranules gastro résistantes d'IPP.

Lorsque la composition se présente est conditionnée sous forme de stick pack, le procédé de préparation comprend en outre une étape de conditionnement dans des sachets individuels. Un tel conditionnement est préféré lorsque la composition est sous forme d'un mélange de poudre et/ou granules et/ou microgranules et/ou liquide et/ou gel.

L'ensemble des molécules, principes actifs, compris dans la composition est donc administré simultanément. Par conséquent, la présente invention permet d'administrer à un patient ladite composition en une seule prise. La présente invention évite donc aux patients de prendre une, deux, voire trois molécules différentes en autant de prises. Bien entendu, l'administration en une prise peut être unique ou séquentielle répétée plusieurs fois au cours de la journée ou de la semaine.

Avantageusement, l'administration simultanée d'alginate et d'IPP permet une meilleure efficacité sur la prévention et/ou le traitement du reflux gastro-oesophagien et par conséquent des vertiges.

Aussi, selon un aspect préféré de l'invention, la composition est destinée à être utilisée comme médicament, préférentiellement dans la prévention et/ou le traitement des vertiges périphériques, des vertiges périphériques récidivants, plus préférentiellement des vertiges positionnels paroxystiques bénins et/ou des vertiges positionnels paroxystiques bénins récidivants.

Selon un autre objet de l'invention, la composition est destinée à être utilisée dans le traitement et/ou la prévention des vertiges dus à une pathologie lithiasique labyrinthique.

Alternativement, la composition selon l'invention peut être utilisée dans la prévention et/ou le traitement du reflux gastro-oesophagien et des vertiges positionnels paroxystiques bénins (VPPB) ou des vertiges périphériques récidivants.

Préférentiellement, la composition selon l'invention est également destinée à être utilisé dans la prévention et/ou le traitement des reflux associés à une gastro-oesophagite spastique.

De par la nature des troubles, très handicapants, il est privilégié une administration orale, ce qui la rend facile, rapide et sans danger pour le patient. Avantageusement, ce mode d'administration permet de s'affranchir de l'intervention d'un professionnel de santé à l'inverse des manipulations de kinésithérapie nécessitant dans certains cas d'être répété afin de soulager le patient. Ainsi, l'administration par voie orale est particulièrement adaptée pour ces pathologies.

Préférentiellement, la dose par prise de la composition selon l'invention pour une administration par voie orale peut comprendre :
- entre 50 mg et 1 g d'alginate, et
- entre 5 mg et 80 mg d'inhibiteur de la pompe à protons oméprazole. Selon un autre objet de l'invention, l'utilisation peut consister à administrer une dose de la composition le matin 30 minutes après le petit déjeuner et une à deux doses de la composition le soir au coucher au moins 3 heures après le repas.

Préférentiellement, la composition pour son utilisation dans le traitement et/ou la prévention des vertiges positionnels paroxystiques bénins consiste à administrer une dose de la composition le matin 30 minutes après le petit déjeuner et deux doses de la composition le soir au coucher au moins 3 heures après le repas.

Alternativement, la composition pour son utilisation dans le traitement et/ou la prévention du reflux gastro-oesophagien et le traitement et/ou la prévention des vertiges positionnels paroxystiques bénins consiste à administrer une dose de la composition le matin 30 minutes après le petit déjeuner et au moins une dose de la composition le soir au coucher au moins 3 heures après le repas.

L'invention est à présent illustrée par des exemples de composition et des résultats d'essais réalisés sur des personnes présentant des vertiges.

### Exemples

### Exemple 1

L'exemple 1 est une composition comprenant un alginate, l'alginate de Sodium, et un inhibiteur de la pompe à protons, l'oméprazole. La composition est composée de :
- 66,7% d'alginate de Sodium,
- 1,3% d'oméprazole, et
- 32% de excipients

Cette composition se présente sous forme de poudre et de microgranules et est obtenue par la mise en oeuvre des étapes suivantes :
- mélange de poudre d'alginate de Sodium et de microgranules d'oméprazole, et
- mélange avec les excipients.

### Exemple 2

L'exemple 2 est une composition comprenant un alginate, l'alginate de sodium, et un inhibiteur de la pompe à protons, l'oméprazole et un antihistaminique H2, la Ranitidine. La composition est composée de :
- 66,7% d'alginate de sodium
- 1,3% d'omépazole,
- 10% de ranitidine, et
- 22% d'excipients.

Cette composition se présente sous forme de poudre et de microgranules et est obtenue par la mise en oeuvre des étapes suivantes :
- mélange de poudre d'alginate de Sodium et de microgranules d'oméprazole, et
- mélange avec des poudres de ranitidine et des excipients.

### Exemple 3 - Résultats d'essais démontrant l'efficacité de l'invention

Patients étudiés dans l'étude ayant reçu une composition comprenant de l'alginate et l'oméprazole selon l'exemple 1.

Les patients inclus dans l'étude sont des personnes des deux sexes présentant des vertiges et des troubles digestifs (RGO, brûlures d'estomac ou gastrite...) confirmés cliniquement ou non et permettant d'établir deux groupes de patients homogènes. Ainsi, le 1^{er} groupe regroupe des patients souffrant de vertiges et de RGO et qui reçoivent un traitement uniquement pour leur vertige tel que bétaSerc ou Tanganil (n=50 patients). Le 2^{ème} groupe de patient regroupe des patients souffrant de vertiges et RGO et qui reçoivent la composition selon l'invention (n=50 patients).

En particulier, le 2^{ème} groupe reçoit un traitement comprenant de l'Omeprazole et de l'Alginate de Sodium. Ce traitement est pris une heure avant le coucher, en l'absence de repas et de boissons dans les trois heures qui précèdent le coucher.

L'efficacité est évaluée après 15 jours de traitement et repose sur une observation clinique, notamment l'absence de vertiges ou non après le début du traitement.

Après analyse des observations cliniques permettant d'établir un diagnostic concernant les vertiges, préférentiellement les vertiges positionnels paroxystiques bénins. Il en ressort les résultats suivants et détaillés dans le tableau 1 ci-dessous.

**[Tableau 1]**

| | bétaSerc ou Tanganil | Composition selon l'invention |
|---|---|---|
| Résultats positifs | 5 | 46 |
| Résultats négatifs | 45 | 4 |

Un résultat est considéré positif lorsque les vertiges ont disparu en moins d'une semaine.

Ainsi, seul 5 patients du 1er groupe ayant reçu le traitement de l'art antérieur (bétaSerc ou Tanganil) ont vu disparaitre les vertiges et sont donc considérés comme traités, soit une efficacité d'environ 10%. En comparaison, 46 patients du 2^{ème} groupe ayant reçu la composition selon l'invention sont considérés comme traités et ne souffrent plus de vertiges, soit une efficacité d'au moins 90%.

### Exemple 4 - Résultats d'essais démontrant l'efficacité de l'invention

### Cas clinique 1

Une première étude a été menée sur un sujet souffrant notamment de vertiges très handicapants. Un premier traitement a été administré à base de médicament anti-nauséeux, de médicaments anti-vertigineux, en particulier l'acétylleucine, la trimétazidine ou la bétahistine, sans effet bénéfique. Un second traitement à base d'inhibiteurs de la pompe à protons et d'alginate de sodium a été administré audit sujet. L'état du sujet s'est alors significativement amélioré et les vertiges ont complétement disparu.

### Cas clinique 2

Une deuxième étude a été mené sur plusieurs patients souffrants notamment de vertiges périphériques. Un traitement à base d'inhibiteurs de la pompe à protons et d'alginate de sodium le soir et matin, a conduit à la suppression des vertiges pour lesdits patients.

### Cas clinique 3

Cette étude a été réalisée sur des patients souffrant de vertiges périphériques. Les patients ont été séparés en deux groupes :
- un premier groupe avec les patients pour lesquels les traitements de référence, en particulier les traitements physiques de kinésithérapie vestibulaire soulage durablement le patient, et
- un deuxième groupe avec les patients pour qui malgré le soulagement immédiat, la récidive était très fréquente et souvent systématique.

Aux patients qui récidivaient leurs vertiges, en complément de la prescription d'un traitement physique, un traitement antiacide a été prescrit, en particulier un inhibiteur de la pompe à proton à 40 mg /jour sur un mois et un alginate de sodium. Après traitement, les patients ne souffraient plus de vertiges et ce sans leur récidive.

## Revendications

1. Composition comprenant :
- au moins un alginate, et
- au moins un inhibiteur de la pompe à protons,
pour son utilisation dans la prévention et/ou le traitement des vertiges positionnels paroxystiques bénins.

2. Composition pour son utilisation selon l'une des revendications précédentes, **caractérisée en ce que** la dose d'alginate par prise est comprise entre 50 mg et 1000 mg et la dose d'inhibiteur de la pompe à proton par prise est comprise entre 5 mg et 80 mg.

3. Composition pour son utilisation selon l'une des précédentes revendications, **caractérisée en ce qu'**elle comprend au moins un inhibiteur de la pompe à protons choisi parmi l'oméprazole, l'esoméprazole, le lansoprazole, le pantoprazole et le rabéprazole.

4. Composition pour son utilisation selon l'une des précédentes revendications, **caractérisée en ce qu'**elle comprend également au moins un anti-acide à action locale et/ou un pansement gastrique.

5. Composition pour son utilisation selon l'une des précédentes revendications, **caractérisée en ce qu'**elle comprend au moins un alginate et de l'oméprazole.

6. Composition pour son utilisation selon l'une des précédentes revendications, **caractérisée en ce qu'**elle se présente sous forme de poudre et/ou de granules et/ou de microgranules.

7. Composition pour son utilisation selon l'une des revendications précédentes, **caractérisée en ce que** les vertiges sont des vertiges dus à une pathologie lithiasique labyrinthique.

8. Composition pour son utilisation selon l'une des revendications précédentes, **caractérisée en ce que** l'utilisation consiste en une administration par voie orale.

9. Composition pour son utilisation selon la précédente revendication, **caractérisée en ce qu'**une dose par prise administrée par voie orale comprend :
- entre 50 mg et 1 g d'alginate, et
- entre 5 mg et 80 mg d'inhibiteur de la pompe à protons.

## Patentansprüche

1. Zusammensetzung, umfassend:
- mindestens ein Alginat, und
- mindestens einen Protonenpumpenhemmer,
zur Verwendung bei der Prävention und/oder der Behandlung von benignen paroxysmalen Lagerungsschwindeln.

2. Zusammensetzung zur Verwendung nach einem der vorstehenden Ansprüche, **dadurch gekennzeichnet, dass** die Dosis von Alginat pro Einnahme zwischen 50 mg und 1000 mg beträgt und die Dosis des Protonenpumpenhemmers pro Einnahme zwischen 5 mg und 80 mg beträgt.

3. Zusammensetzung zur Verwendung nach einem der vorstehenden Ansprüche, **dadurch gekennzeichnet, dass** sie mindestens einen Protonenpumpenhemmer umfasst, der aus Omeprazol, Esomeprazol, Lansoprazol, Pantoprazol und Rabeprazol ausgewählt ist.

4. Zusammensetzung zur Verwendung nach einem der vorstehenden Ansprüche, **dadurch gekennzeichnet, dass** sie auch mindestens eine Anti-Säure mit lokaler Wirkung und/oder ein Magenpflaster umfasst.

5. Zusammensetzung zur Verwendung nach einem der vorstehenden Ansprüche, **dadurch gekennzeichnet, dass** sie mindestens ein Alginat und Omeprazol umfasst.

6. Zusammensetzung zur Verwendung nach einem der vorstehenden Ansprüche, **dadurch gekennzeichnet, dass** sie in Form von Pulver und/oder Granulaten und/oder Mikrogranulaten vorliegt.

7. Zusammensetzung zur Verwendung nach einem der vorstehenden Ansprüche, **dadurch gekennzeichnet, dass** die Schwindel Schwindel aufgrund einer Labyrinthlithiasiserkrankung sind.

8. Zusammensetzung zur Verwendung nach einem der vorstehenden Ansprüche, **dadurch gekennzeichnet, dass** die Verwendung aus einer Verabreichung auf oralem Weg besteht.

9. Zusammensetzung zur Verwendung nach dem vorstehenden Anspruch, **dadurch gekennzeichnet, dass** eine Dosis pro Einnahme, die auf oralem Weg verabreicht wird, umfasst:
- zwischen 50 mg und 1 g Alginat, und
- zwischen 5 mg bis 80 mg Protonenpumpenhemmer.

## Claims

1. Composition, comprising:
- at least one alginate, and
- at least one proton pump inhibitor,
for use in the prevention and/or treatment of benign paroxysmal positional vertigo.

2. Composition for use according to the preceding claim, **characterized in that** the dosage of alginate per dose is between 50 mg and 1000 mg, and the dosage of proton pump inhibitor per dose is between 5 mg and 80 mg.

3. Composition for use according to either of the preceding claims, **characterized in that** it comprises at least one proton pump inhibitor selected from omeprazole, esomeprazole, lansoprazole, pantoprazole and rabeprazole.

4. Composition for use according to any of the preceding claims, **characterized in that** it also comprises at least one local-action anti-acid and/or a gastric dressing.

5. Composition for use according to any of the preceding claims, **characterized in that** it comprises at least one alginate and omeprazole.

6. Composition for use according to any of the preceding claims, **characterized in that** it is in the form of powder and/or granules and/or microgranules.

7. Composition for use according to any of the preceding claims, **characterized in that** the vertigo is vertigo due to a labyrinthine lithiasis disorder.

8. Composition for use according to any of the preceding claims, **characterized in that** the use consists of oral administration.

9. Composition for use according to the preceding claim, **characterized in that** an orally administered dosage comprises per dose:
- between 50 mg and 1 g alginate, and
- between 5 mg and 80 mg proton pump inhibitor.
